# FASCICULE DE BREVET EUROPEEN

(11) **EP 0 436 013 B1**
(45) Date de publication et mention de la délivrance du brevet: **01.06.1994**
(21) Numéro de dépôt: 90912262.4
(22) Date de dépôt: 26.07.1990
(51) Int. Cl.: A41D 13/00, A61F 5/00

(54) **APPAREIL PREVENTIF DES FRACTURES DU COL DU FEMUR POUR LES PERSONNES AGEES**
VORRICHTUNG ZUM VERMEIDEN VON SCHENKELHALSFRAKTUREN ÄLTERER PERSONEN
DEVICE FOR PREVENTING FRACTURES OF THE NECK OF THE FEMUR IN ELDERLY PEOPLE

(30) Priorité: 26.07.1989 FR 8910036
(43) Date de publication de la demande: 10.07.1991
(73) Titulaire: CHARPENTIER, Pierre, 91190 Gif-sur-Yvette (FR)
(72) Inventeur: CHARPENTIER, Pierre, 91190 Gif-sur-Yvette (FR)
(74) Mandataire: Debay, Yves
(86) Numéro de dépôt international: FR9000568
(87) Numéro de publication internationale: WO9101658

(56) Documents cités:
- DE-A- 3 616 890
- FR-A- 96 149
- FR-A- 2 120 500
- GB-A- 1 479 733
- GB-A- 1 514 540
- US-A- 4 270 527
- US-A- 4 413 620
- US-A- 4 737 994

## Description

La présente invention concerne un appareil préventif des fractures du col du fémur pour les personnes agées.

Avec l'âge, la résistance mécanique des os du squelette humain décroit et peut, dans certains cas, conduire à une fragilisation générale. Cette fragilisation, combinée avec une stabilité moins grande de la personne en position debout, avec par conséquent des risques de chutes plus fréquentes, peut entraîner de nombreuses fractures, en particulier celle du col du fémur.

Malgré les progrès de la chirurgie, la réduction de ces fractures reste un traitement important, entraînant une hospitalisation de plusieurs semaines, pour une bonne part en position couchée, d'où de lourdes conséquences qui sont :
- une atrophie musculaire nécessitant une rééducation à résultat en général bien souvent partiel, compte tenu de l'âge du patient, d'où
- une réduction des capacités motrices, et
- une appréhension accrue liée à la crainte d'un nouvel accident.

En conséquence, l'accidenté voit son autonomie et son activité diminuer.

Par ailleurs, le traitement de ces fractures est très onéreux, par suite, en particulier, de la durée de l'hospitalisation et de la rééducation.

L'intérêt d'un appareil préventif est donc aisément perçu, tant sur le plan humain que sur le plan économique.

Un tel appareil est connu par le brevet US 4 737 994 qui décrit un appareil préventif des fractures du col du fémur pour les personnes agées comprenant un élément support fixé de façon amovible, d'éléments de protection gonflables, l'élément support étant fixé à la taille, les éléments gonflables étant constitués par une pluralité d'éléments soufflets qui une fois gonflés prennent une forme de boudin, ces éléments gonflables étant solidaires de l'élément support et adjacents entre eux de façon à couvrir en permanence une zone s'étendant substantiellement de la taille jusqu'à mi-cuisse.

Toutefois, le vêtement enseigné doit être porté par l'utilisateur en permanence gonflé pour qu'il puisse remplir son rôle. Ceci est particulièrement inesthétique pour l'utilisateur qui aura tendance à ne pas gonfler du tout l'appareil ou le gonfler partiellement, réduisant ainsi son efficacité.

Par ailleurs, il est connu des systèmes de protection contre les chutes constitués d'une structure gonflable enfermée dans un container et fixée sur une personne ou un objet qui, lors de la détection d'une diminution importante de l'accélération induite par une chute provoque le gonflement d'une enveloppe déclenchant la rupture du container et le déploiement de cette enveloppe autour de la personne ou de l'objet, jusqu'à ce que la personne ou l'objet soient totalement enveloppés.

Une telle structure nécessite également un parachute permettant d'orienter et de maintenir l'ensemble dans une position préférentielle assurant ainsi une reprise de contact avec la terre ferme par les pieds ou le dos.

Un tel système s'applique à des personnes susceptibles de chuter d'une grande hauteur et protège l'ensemble du corps par enveloppement, et implique un équipement d'une étanchéité parfaite et capable de se déployer à coup sûr pour envelopper totalement la personne. Par ailleurs, pour éviter les déclenchements intempestifs du système lors des activités des personnes à protéger travaillant à de grandes hauteurs, il est nécessaire d'avoir un système de déclenchement, se déclenchant pour des valeurs d'accélération relativement faibles de façon à éviter que lors des activités normales, les personnes utilisant ce système de protection se trouvent subitement enveloppées, ce qui dans le cas de travailleurs de l'industrie du bâtiment, pourrait provoquer leur chute.

Ces deux raisons elles-mêmes suffisent à faire comprendre que la mise en action d'un tel système est suffisamment longue pour être inadaptée au but de l'invention.

Un premier but de l'invention est donc de concevoir un appareil préventif des fractures du col du fémur pour les personnes agées qui soit d'utilisation aisée et de mise en action rapide de telle sorte que le gonflage ait lieu avant le contact avec le sol permettant une utilisation permanente du dispositif.

Ce but est atteint par le fait que l'appareil préventif de fractures du col du fémur pour les personnes agées, comprenant un élément support fixé de façon amovible, d'éléments de protection gonflables, l'élément support étant fixé à la taille; les éléments gonflables étant constitués par une pluralité d'éléments à soufflets, qui une fois gonflés, prennent une forme de boudin, ces éléments gonflables étant solidaires de l'élément support et adjacents entre eux de façon à couvrir en permanence une zone s'étendant substantiellement de la taille jusqu'à mi-cuisse; caractérisé en ce que :
- l'appareil est muni de moyens de glonflage comprenant des capsules de gonflage comportant une amorce pyrotechnique à déclenchement électrique, les moyens de gonflage effectuant le gonflage de la pluralité des éléments gonflables en un temps inférieur à 0,3 secondes;
- des moyens de déclenchement du gonflage et de détection des conditions d'une chute sont disposés sur le devant de l'abdomen, les moyens de déclenchement du gonflage comprenant un contact électrique mettant en relation ou non une source électrique avec chacune des amorces pyrotechniques des capsules de gonflage des éléments gonflables et les moyens de détection des conditions d'une chute étant constitués d'un accéléromètre se déclenchant à partir d'un seuil d'accélération compris entre 0,1 et 0,4 g, cet accéléromètre commandant le contact électrique pour mettre en relation ou non la source électrique avec chacune des amorces pyrotechniques des capsules de gonflage des éléments gonflables.

Selon une autre caractéristique de l'invention, l'appareil comporte une temporisation de déclenchement des moyens de gonflage de l'ordre de 0,2 seconde et en ce que les moyens de gonflage effectuent le gonflage en un temps inférieur à 0,1 seconde.

Selon une autre particularité de l'invention, les éléments gonflables comportent chacun une soupape de sécurité à fort débit.

Selon une autre particularité, chaque soupape à fort débit est constituée d'une fente dans un matériau plastique dont un bord a une forme de jonc cylindrique et l'autre une forme de gorge cylindrique.

Selon une autre particularité, les éléments gonflables forment une jupe.

Selon une autre particularité, les éléments gonflables couvrent la partie arrière du bassin jusqu'au bas des muscles fessiers et sur le côté des hanches descendent jusqu'à mi-cuisse.

Selon une autre particularité, les éléments gonflables descendant jusqu'à mi-cuisse sont reliés entre eux par un élément de fixation à la cuisse.

Selon une autre particularité, les éléments gonflables constituent un cuissard ouvert sur la partie avant basse du bassin.

Selon une autre particularité, chaque élément gonflable comporte une capsule de gonflage amovible, comportant dans une enveloppe un premier compartiment communiquant avec l'élément gonflable par un opercule et contenant un gaz comprimé ou liquéfié et contenant une charge de poudre pyrotechnique mise à feu par une amorce pyrotechnique.

Selon une autre particularité, la charge de poudre pyrotechnique est placée dans un second compartiment communiquant avec le premier par un bouchon à faible résistance.

Un autre but de l'invention est de proposer une autre variante de l'appareil.

Ce but est atteint par le fait que l'appareil préventif de fractures du col du fémur pour les personnes agées, comprenant un élément support, fixé de façon amovible ; d'éléments de protection gonflables, l'élément support étant fixé à la taille ; les éléments gonflables étant constitués par une pluralité d'éléments à soufflets, qui, une fois gonflés, prennent une forme de boudin, ces éléments gonflables étant solidaires de l'élément support et adjacents entre eux de façon à couvrir en permanence une zone s'étendant substantiellement de la taille jusqu'à mi-cuisse ; caractérisé en ce que :
- l'appareil est muni de moyens de gonflage comprenant une enceinte contenant un gaz comprimé, les moyens de gonflage effectuant le gonflage de la pluralité des éléments gonflables en un temps inférieur à 0,3 seconde ;
- des moyens de déclenchement du gonflage et de détection des conditions d'une chute sont disposés sur le devant de l'abdomen, les moyens de déclenchement du gonflage comprenant un percuteur ou une électrovanne destiné à libérer un gaz comprimé enfermé dans l'enceinte et les moyens de détection des conditions d'une chute étant constitués d'un accéléromètre se déclenchant à partir d'un seuil d'accélération compris entre 0,1 et 0,4 g, cet accéléromètre commandant le percuteur ou l'électrovanne de façon à libérer le gaz comprimé enfermé dans l'enceinte.

Un autre but de l'invention est de prévoir un appareil préventif qui puisse être porté en permanence par l'utilisateur.

Ce but est atteint par le fait que l'appareil comporte une doublure à l'intérieur de l'élément support, amovible ou non, et en matériau confortable.

Selon une autre caractéristique, l'appareil est incorporé et rendu solidaire de façon amovible ou non de vêtements.

Selon une autre particularité, l'appareil est équipé d'un contact électrique actionnable manuellement pour condamner temporairement la commande par le détecteur de seuil d'accélération du contact électrique mettant en relation une source électrique avec chacune des amorces pyrotechniques à déclenchement électrique des capsules de gonflage des éléments gonflables.

Selon une autre particularité, le contact électrique est constitué par une des agrafes permettant de boucler l'élément support autour de la taille.

Selon une autre particularité, l'appareil est équipé d'un contact électrique détectant les inclinaisons du corps supérieures à 60°, lequel contact électrique condamne la commande par le détecteur de seuil d'accélération du contact électrique mettant en relation une source électrique avec chacune des amorces pyrotechniques à déclenchement électrique des capsules de gonflage des éléments gonflables.

Un dernier but de l'invention est de proposer un procédé de prévention des fractures du col du fémur.

Ce but est atteint par le fait que le procédé utilise un élément gonflable pour
- couvrir en permanence par l'intermédiaire de l'élément gonflable une zone s'étendant substantiellement de la taille jusqu'à mi-cuisse et consiste à :
- détecter les conditions d'une chute ;
- retarder le déclenchement du gonflage de l'ordre de 0,2 seconde ;
- gonfler l'élément en un temps inférieur à 0,1 seconde;
- utiliser la surpression due au contact avec la surface pouvant occasionner la fracture pour déclencher une soupape de sécurité prévue sur chaque élément gonflable dont le rôle est de déclencher pour une valeur relativement précise de la pression et d'assurer un fort débit instantané pour des pressions différentielles modérées.

D'autres caractéristiques et avantages de la présente invention apparaîtront plus clairement à la lecture de la description ci-après faite en référence aux dessins annexés dans lesquels :
- la figure 1A représente une vue de face d'une première variante de réalisation de l'appareil préventif;
- la figure 1B représente une vue de dos de cette première variante de l'appareil préventif;
- la figure 2A représente une vue de face d'une deuxième variante de l'appareil préventif;
- la figure 2B représente une vue de dos de cette deuxième variante de l'appareil préventif;
- la figure 3 représente un mode de réalisation d'un détecteur de seuil d'accélération;
- la figure 4A représente un mode de réalisation d'un détecteur d'inclinaison et la figure 4B le détecteur en position de détection;
- la figure 5 représente le schéma électrique de connexion des moyens de déclenchement et de gonflage de l'appareil;
- la figure 6 représente un élément de gonflage amovible de l'appareil;
- la figure 7 représente l'appareil en service lors d'une chute ;
- la figure 8 représente un deuxième mode de réalisation d'un élément de gonflage de l'appareil ;
- la figure 9 représente un diagramme temporel des différents paramètres intervenant dans le fonctionnement de l'appareil ;
- la figure 10 représente une vue en coupe d'une soupape de sécurité.

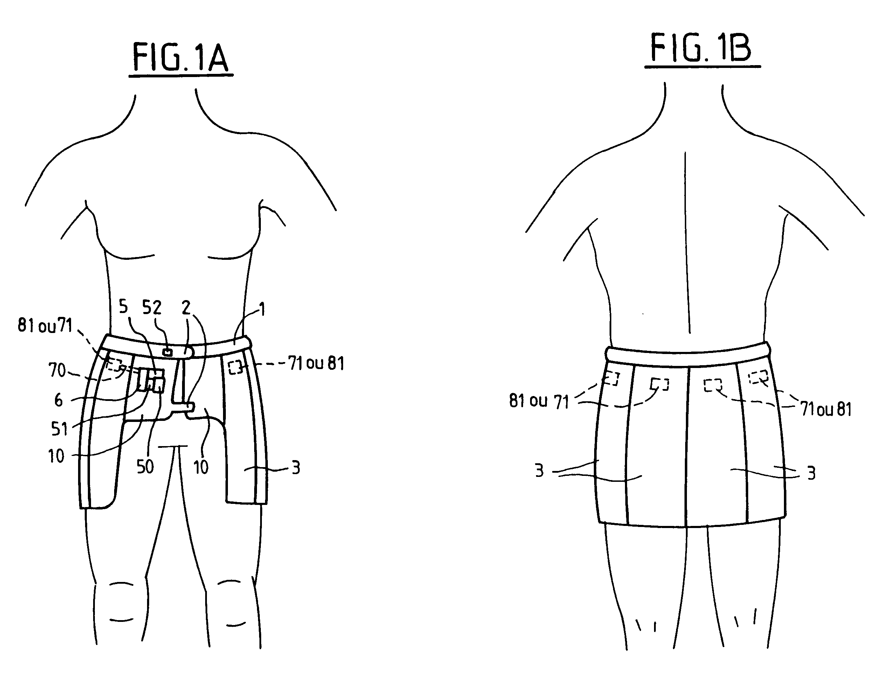

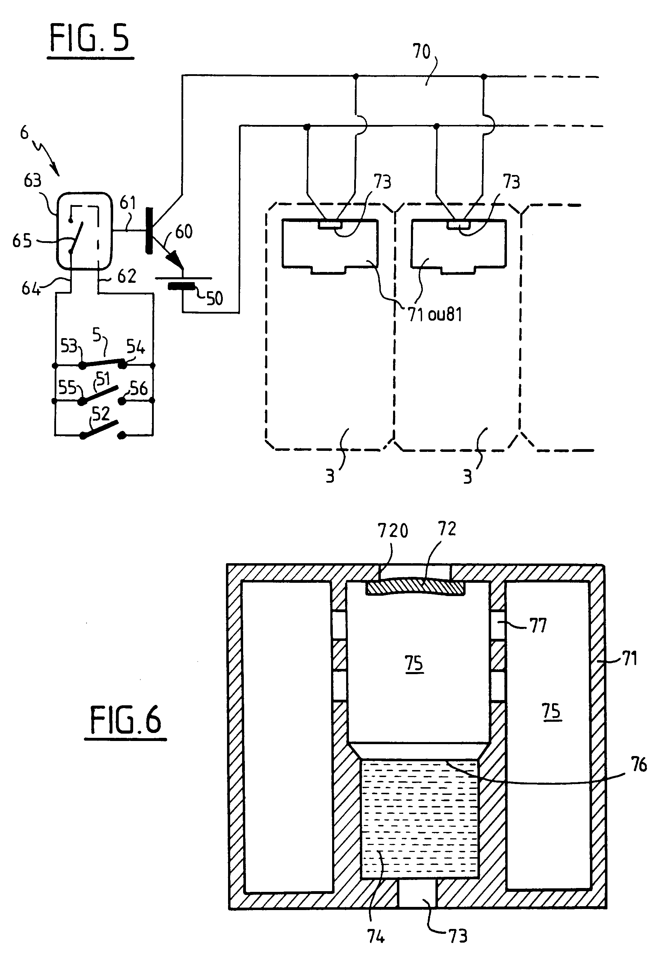

L'appareil est constitué, comme on peut le voir à la figure 1A, par un élément de support formé d'une ceinture (1) et d'une partie en forme de jupe (10) destinées à envelopper totalement l'os du bassin et les muscles fessiers ainsi que la partie haute des cuisses. Cet élément (10) comporte vers l'extérieur des éléments gonflables solidaires et formés, comme on peut le voir à la figure 1B, d'une succession de boudins longitudinaux (3) adjacents en matériau étanche au gaz de gonflage. Ces boudins sont disposés, par exemple, de façon parallèle à l'axe de symétrie de la colonne vertébrale et des membres inférieurs. Ces boudins gonflables peuvent être formés d'éléments à soufflets qui, dans leur état non gonflé, occupent un minimum d'épaisseur. Les boudins gonflables (3) solidaires de la partie support (10) sont disposés et ont des dimensions telles qu'elles permettent de former une sorte de jupe enveloppant totalement la partie arrière du corps et descendant suffisamment bas pour protéger à la fois le coccyx, l'os iliaque et le col du fémur. La partie avant de l'appareil ne comporte pas d'éléments gonflables sur toute la partie qui se trouve en vis-à-vis de l'abdomen et de la partie basse de l'abdomen. Un système d'agraffes de fixation (2) permet de maintenir la ceinture (1) et de maintenir également le support (10) sur le corps de l'utilisateur. Le dispositif comporte en outre des moyens (71,81) de gonflage de chaque boudin déclenchables par exemple électriquement et reliés par une liaison électrique (70) à un dispositif de déclenchement (6) comportant éventuellement une temporisation, associé à un accéléromètre (5), à une pile électrique (50), à un dispositif de détection d'inclinaison (51), à un interrupteur (52) de sécurité constitué par les agraffes de fermeture de la ceinture pour mettre le système hors service lorsqu'on enlève la ceinture et une temporisation, comme on le verra en liaison avec la figure 5.

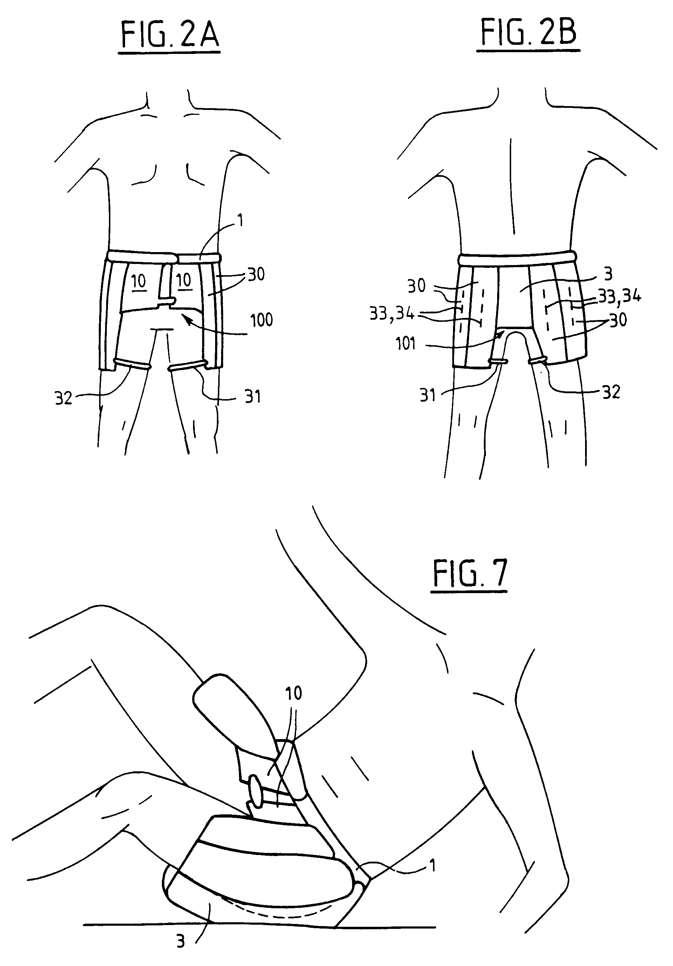

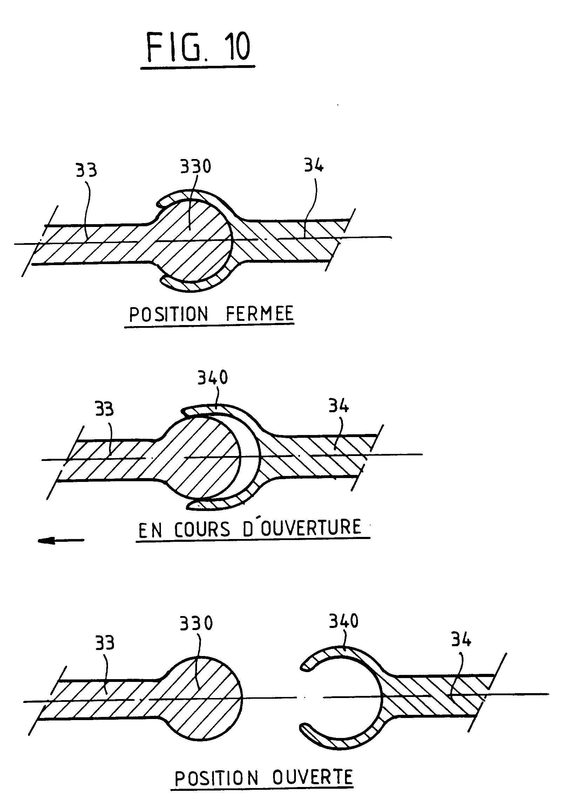

Les figures 2A et 2B représentent une deuxième variante de réalisation de l'élément de support et des éléments gonflables. Dans cette variante, l'élément de support et les éléments gonflables ont substantiellement la forme d'un cuissard dans lequel les parties avant et arrière situées au niveau de l'entrejambe auraient été supprimées. Ainsi l'élément (10) conserve sa forme en jupe mais les éléments gonflables (30), situés sur les hanches et sur les côtés le long des cuisses ont des longueurs plus importantes que les éléments gonflables (3) situés au voisinage de la partie centrale des muscles fessiers. Cette différence de longueur dans les éléments gonflables (3) crée sur la partie arrière une échancrure (101) en forme de U dont les branches verticales sont orientées vers le bas et sur la partie avant de l'appareil une deuxième échancrure (100) également en forme de U dont les branches sont également orientées vers le bas. Les extrémités des éléments gonflables (30), de longueur plus importante que les éléments gonflables (3) situés au niveau des muscles fessiers sont rendues solidaires de bandes de fixation (31, 32) permettant la fixation et le maintien en place le long des cuisses de ces éléments. Les éléments de fixation (31, 32) peuvent être du type utilisant des fermetures connues sous la marque "velcro" ou tout autre système à boucles, à pressions ou à élastique. Les éléments gonflables (30) sont pourvus chacun de soupapes (33,34) de sécurité à fort débit. Ces soupapes (33,34) doivent remplir les conditions suivantes :
- déclencher pour une valeur relativement précise de la pression, autour de 0,5 bar relatif :
- assurer un fort débit instantané compris entre 100 et 150 litres de gaz par seconde, pour des pressions différentielles modérées (0,3 à 0,5 bar). Il en résulte une section de passage très importante, de l'ordre d'une dizaine de centimètres carrés ;
- être facilement réarmable après déclenchement ;
- être facilement ajustable en débit, on peut en effet utiliser ce paramètre pour règler l'appareil en fonction du poids du porteur ;
- ne pas éjecter de partie solide lors du déclenchement ;
- être légères et peu encombrantes ;
- être bon marché ;
et sont réalisées de la façon représentée à la figure 10.

Parmi les diverses solutions, celle de la figure 10 remplit toutes les conditions ci-dessus.

Il s'agit de deux bandes continues (33,34) dont l'une (33) comporte sur un bord un jonc cylindrique (330) et l'autre (34) sur son bord une gorge cylindrique ouverte (340) et dont les bords s'emboîtent lors de la fermeture. Le matériau est de matière plastique déformable. Sans être parfaitement étanche, ce système, en position fermée, présente un débit de fuite tout à fait négligeable, eu égard aux volumes en cause et au temps de fonctionnement de l'appareil. Si ce type de fermeture est soumis à une pression différentielle, il se crée une force tendant à écarter les deux bandes (33,34). Lorsque la pression croit, cette force va augmenter, déformer les bords de la gorge (340) et dépasser la résistance de l'assemblage des bords (330,340) pour provoquer l'ouverture en un endroit. Une fois ce processus engagé, il va immédiatement se transmettre à toute la longueur de la fermeture, du fait de la forme des bandes.

En choisissant une matière plastique ayant de bonnes caractéristiques élastométriques, variant peu avec le vieillissement, ce qui semble être déjà le cas pour les fabrications actuelles, on disposera d'un système qui réagira à une pression différentielle suffisamment constante et très facilement réarmable.

Le débit est facilement ajusté, il suffit de modifier la longueur d'ouverture. Pour ce faire, il suffit de condamner une partie de la longueur par des clips métalliques sertis aux extrémités.

Enfin, cette soupape de sécurité ne pèse que quelques grammes et est très bon marché.

L'ajout d'une soupape de décharche à fort débit a comme avantage d'assurer un meilleur amortissement de la chute, de limiter la pression dans les "boudins" de gonflage qui permet donc l'utilisation d'un tissu plus léger et plus souple, d'éviter les risques de rebondissement après amortissement et d'assurer un dégonflage presque immédiat évitant d'encombrer le porteur à la fin de la chute.

La figure 5 représente le schéma électrique du dispositif de déclenchement des moyens de gonflage, constitué par des capsules de gonflage formées dans une enveloppe métallique (71) comme représenté à la figure 6. Ces capsules de gonflage (71) comportent une amorce électro-pyrotechnique (73) permettant la mise à feu de chaque capsule lorsqu'un courant électrique circule dans les fils de liaison (70) reliant chaque amorce électrique aux deux bornes d'une pile électrique (50) sous certaines conditions commandées par le circuit de déclenchement (6) associé à un accéléromètre (5).

L'accélération étant une grandeur vectorielle, les accéléromètres sont directionnels. Il faudrait théoriquement prévoir 3 accéléromètres disposés orthogonalement et sommer leurs mesures pour connaître la vraie valeur d'accélération. En fait un seul accéléromètre est suffisant et ceci permet de réduire le coût du dispositif électronique.

Les chutes se produisant normalement depuis la position debout, la diminution brutale d'accélération au niveau du corps est alors mesurée intégralement.

L'appareil détecte également les chutes avec le corps en position inclinée, et ce au moins jusqu'à soixante degrés. Pour un tel angle, la composante verticale de l'accélération est encore de 0,5 g, soit bien au-dessus du seuil de déclenchement de 0,2 g indiqué plus haut. Lors d'une chute, le détecteur de seuil d'accélération fonctionne lorsque la composante de l'accélération dans sa direction n'est plus que de 0,2 g, correspondant à 0,4 g d'accélération totale.

Cependant, et pour éviter les fonctionnements intempestifs, il faut placer le détecteur de seuil d'accélération au dessous de la zone de flexion de la taille, soit à mi-chemin entre le pubis et la ceinture.

Dans cette configuration, il y a risque de déclenchement intempestif si le porteur se couche avec l'appareil. Pour éviter cet inconvénient, il suffit d'y coupler un interrupteur (51) à mercure qui annulera la détection de chute lorsque l'inclinaison dépassera 60°.

De plus, lorsque l'appareil est détaché du corps, sa manipulation pourrait entraîner le déclenchement intempestif du gonflage. Ce risque peut être éliminé en associant à une des agraffes de fermeture un interrupteur (52) de sécurité mettant le système de détection de chute hors service lorsque la gaine est dégraffée.

La pile électrique (50) a sa borne négative reliée d'une part à un des deux fils (70) de liaison à l'amorce pyrotechnique (73) et, d'autre part sa borne positive à l'émetteur d'un transistor NPN (60) dont le collecteur est relié à l'autre fil (70) de laison à l'amorce pyrotechnique (73). Cet interrupteur électronique est commandé par un circuit (63) comportant éventuellemnt une temporisation (65) de l'ordre de 0,2 seconde. Le temporisateur (65), règlé par exemple entre 10 et 20/100ème de seconde, permet d'éviter les déclenchements intempestifs en cas de mouvement brutal très limité, tel que de se laisser tomber sur un siège dans la toute dernière phase du mouvement de la position debout à la position assise. En effet, les observations du comportement des personnes agées ont montré qu'elles opéraient fréquemment de cette manière, leurs muscles n'étant plus capables de retenir le poids du corps à partir d'une certaine flexion des genoux et des hanches. Ceci implique un temps de gonflage réduit à 0,1 ou 0,15 seconde.

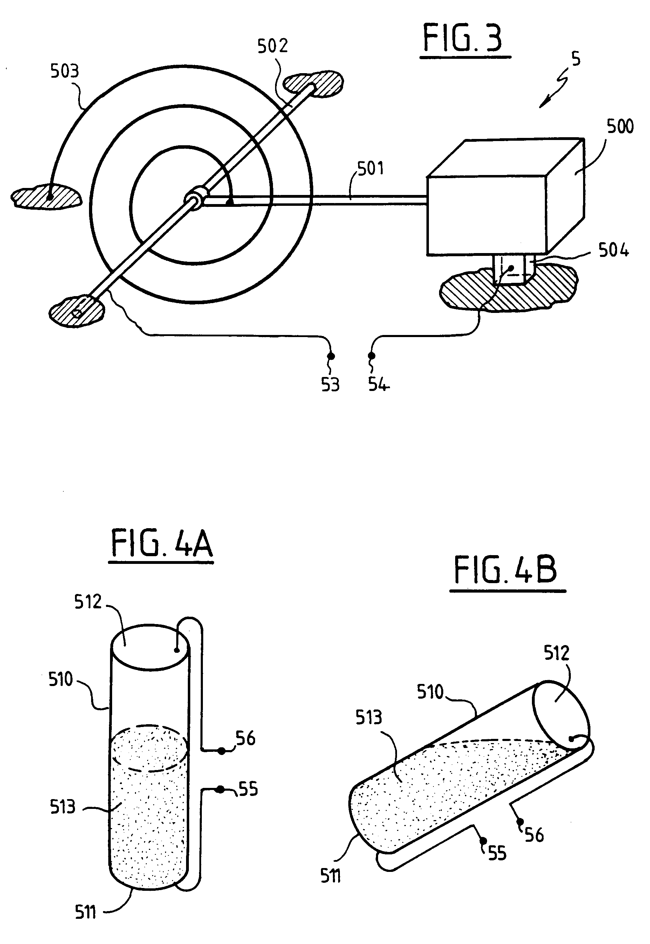

Ce circuit (63) est branché, par la sortie (61) soit sur la base, pour un transistor NPN, ou la grille, pour un transistor CMOS, de l'interrupteur électronique (60). Le circuit (63) permet la détection de l'ouverture du circuit constitué par l'ensemble formé par le contact de l'accéléromètre (5) fermé au repos, le contact (51) du détecteur d'inclinaison ouvert au repos, le contact (52) d'ouverture de la ceinture ouvert au repos, branchés en parallèle aux entrées (62,64) de ce circuit. La sortie (61) du circuit (63) est activée après le délai de temporisation de 0,2 seconde lorsque le circuit des contacts (5,51,52) est ouvert. Les interrupteurs (51,52) correspondent à des dispositifs de sécurité et sont montés en parallèle avec l'interrupteur (5). Ils sont normalement ouverts et ne s'opposent alors en rien au fonctionnement en cas de détection de chute. Si une sécurité est actionnée, l'interrupteur correspondant (52,51) se ferme, court-circuitant l'interrupteur du détecteur de seuil et le rendant inopérant. L'interrupteur (51) peut être un détecteur d'inclinaison, tel que celui décrit ci-après en liaison avec la figure 4, tandis que l'interrupteur (52) peut être constitué par celui associé à l'une des agraffes de fermeture de la ceinture pour mettre le dispositif hors service lorsque l'appareil est dégraffé. L'interrupteur (5) représente, soit un accéléromètre détectant le dépassement d'un seuil d'accélération et déclenchant, en fonction de ce dépassement le déplacement du contact mobile de l'interrupteur (5); soit un détecteur de seuil d'accélération simplifié, tel que celui décrit ci-après en liaison avec la figure 3.

Dans ce détecteur, une masselotte (500) de masse (m) est fixée en bout d'une tige (501) dont l'autre extrémité est solidaire d'un axe (502). L'ensemble est monté dans un plan horizontal et un ressort à spirale (503) est fixé entre un point fixe et un point de la tige (501), de façon à exercer une force de rappel pouvant entraîner un mouvement dans le sens inverse des aiguilles d'une montre. Au repos la masselotte (500) repose sur une butée (504) reliée électriquement à la borne (54). Le circuit électrique est constitué par la butée (504), la masselotte (500), la tige conductrice (501) et l'axe conducteur (502) pour enfin sortir par la borne (53) qui est reliée, comme on peut le voir à la figure 5, au circuit de commande (63) de l'interrupteur électronique (60) du circuit de déclenchement (6). Le détecteur de seuil d'accélération est fixé sur la gaine de protection (10), de telle sorte qu'il soit en position horizontale lorsque le porteur est en position debout. Lors des mouvements normaux, les accélérations induites sont telles que l'accélération verticale reste supérieure à 0,2 g, le ressort est règlé pour exercer une traction de 0,2 g, g étant l'accélération de la pesanteur. La masselotte (500)reste donc sur sa butée et la continuité électrique reste assurée. En cas de chute l'accélération verticale baisse instantanément en dessous de 0,2 g et la force de traction exercée vers le haut par le ressort prévaut sur la force induite par la masse (m). La masselotte (500) s'élève en se détachant de la butée (5) interrompant ainsi la continuité électrique du circuit. Ce phénomène est détecté par le circuit détecteur d'impulsion électrique (6) qui commande par le circuit (63) la mise en conduction de l'interrupteur électronique (60).

Dans une autre variante le détecteur peut être réalisé par un ressort de rappel vertical relié directement à la masselotte ou bien par un ressort à lames remplaçant la tige (501) et le ressort à spirale (503).

Le détecteur (51) de prise de position allongée est constitué par une enveloppe cylindrique étanche et isolante (510) à l'exception du fond (511) et du couvercle (512) qui servent d'électrode et sont chacun reliés par un fil électrique aux bornes respectives (55,56). Cette enveloppe (510) est partiellement remplie de mercure (513). L'ensemble est installé sur la gaine de protection (10) de telle sorte qu'il soit sensiblement vertical lorsque le porteur est en position debout. Dans ces conditions le mercure (513) est uniquement en contact avec l'électrode inférieure (511) et il n'y a pas continuité électrique entre les bornes (55,56). Quand l'inclinaison atteint une valeur de l'ordre, par exemple, de 60°, le mercure atteint l'électrode (512) tout en restant en contact avec l'électrode de fond (511). Il y a alors continuité électrique entre les bornes (55,56) ce qui correspond à la fermeture de l'interrupteur (51). Dans ce cas, une ouverture du contact (5) correspondant à une détection d'accélération ne sera pas prise en compte par le circuit électronique (6) de commande de l'interrupteur électronique (60,61).

Dans la réalisation de la figure 5, la ceinture comporte un ensemble de plusieurs éléments gonflables formé de boudins longilignes dont chacun comporte une capsule de gonflage (71,81) avec sa propre amorce électrique, l'ensemble étant monté de façon étanche ou quasi-étanche.

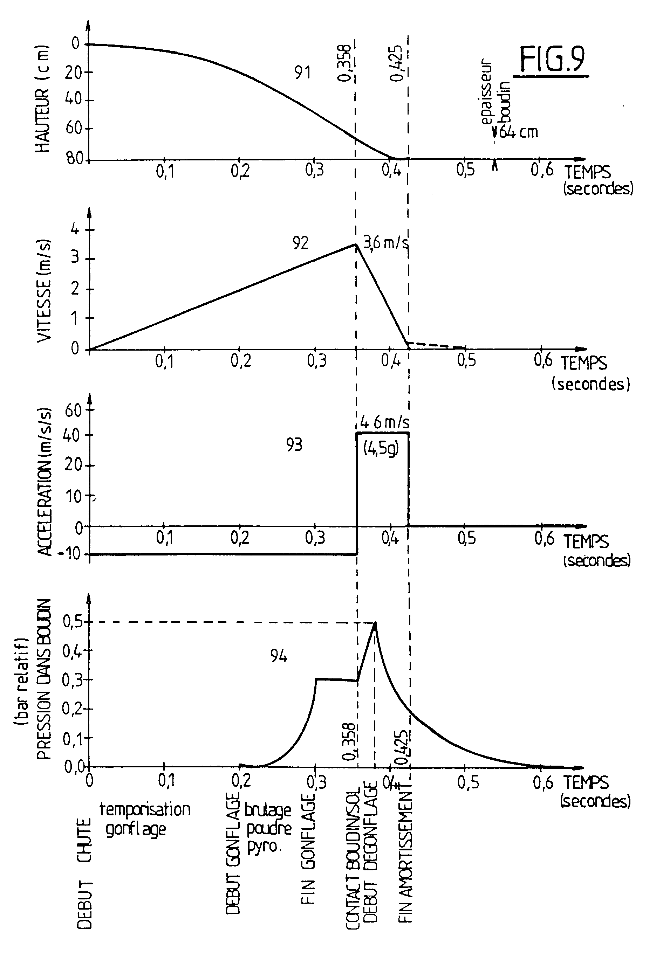

Un tel dispositif permet d'assurer un gonflage rapide de la ceinture en fonction du déroulement de la chute, comme représenté à la figure 9. Nous rappelons que le gonflage doit s'effectuer en un temps inférieur à 0,3 seconde, avec le délai de temporisation inclu, pour permettre une protection efficace lors des chutes des personnes de leur position verticale vers une position horizontale.

Les principaux paramètres caractérisant la chute sont représentés sur les courbes de la figure 9. Le déplacement vertical est représenté en fonction du temps (91). Dans une première phase, le corps, soumis à l'accélération de la pesanteur g (10 m/s/s), chute suivant un mouvement uniformément accéléré si l'on considère que la hauteur des hanches est 0,80 m et l'épaisseur des boudins gonflés 0,16 m. Les boudins gonflés entre temps atteignent le sol après une chute de 0,64 m (0,80 m - 0,16 m) après 0,36 seconde. Ensuite commence l'amortissement qui dure environ 0 ,065 seconde. La durée totale de processus est de 0,425 seconde. Après cette période, le gonflage des boudins est plus néfaste qu'utile.

La courbe (92) représente la vitesse nulle au début de la chute. Cette vitesse croit linéairement jusqu'au contact des boudins avec le sol où elle atteint 3,6 m/seconde, soit 13 km/heure. Elle décroit ensuite rapidement, et approximativement linéairement pendant l'amortissement à la fin duquel elle est évidemment nulle. L'accélération représentée en fonction du temps par la courbe (93) est, dans la phase chute, celle de la pesanteur (g). De même, l'accéléromètre détectant une variation brutale d'accélération doit être suffisamment sensible pour permettre un déclenchement pour des accélérations de l'ordre de 0,1 à 0,4 g permettant ainsi un gonflage du dispositif avant le contact avec le sol de la personne. On prendra de préférence 0,2 g. Ce seuil de 0,1 à 0,4 g est par contre suffisamment élevé pour permettre à une personne se déplaçant normalement par ses propres moyens de ne pas déclencher de façon intempestive le gonflage. Pendant la phase d'amortissement, elle est de l'ordre de 5 g. L'accélération redevient ensuite nulle puisque l'ensemble soumis à l'accélération de la pesanteur est au sol.

La pression relative représentée en fonction du temps par la courbe (94) est évidemment nulle avant le gonflage. Après 0,2 seconde, le processus de gonflage est initié et après environ un dixième de seconde, les boudins sont gonflés à 0,3 bar relatif. Cette pression reste stable jusqu'au contact des boudins avec le sol. La pression monte alors rapidement jusqu'à 0,5 bar relatif en temps de 1 à 2 centièmes de seconde. A cette pression, une soupape de décharge (33,34) s'ouvre, provoquant un dégonflage progressif (en tous cas plus rapide que l'écrasement des boudins dans la phase amortissement). La pression qui chute rapidement, est encore de l'ordre de 0,2 bar relatif à la fin de l'amortissement, et assez voisine de zéro après environ 2 dixièmes de seconde. L'évolution de la pression telle que décrite ci-dessus assure un amortissement suffisamment progressif d'une part, et prévient tout risque de rebondissement d'autre part.

Pour permettre une utilisation avec des déclenchements multiples, les capsules de gonflage sont montées de façon amovible sur les boudins formant les éléments gonflables dans le cas de la variante correspondant à la figure 5 où chaque élément gonflable comporte une capsule.

L'étanchéité de chaque élément gonflable comportant une capsule, sera réalisée par le serrage de l'élément gonflable sur la périphérie cylindrique du boitier (71) de la capsule. Chaque capsule de gonflage est constituée d'un élément formant une enveloppe métallique (71) percée à une extrémité par un orifice (720) provisoirement fermé par un opercule (72) et à une autre extrémité par un orifice provisoirement fermé par une amorce pyrotechnique à déclenchement électrique (73). Cette amorce pyrotechnique à déclenchement électrique (73) débouche dans un compartiment rempli d'une poudre pyrotechnique (74). Ce compartiment (74) communique avec un autre compartiment (75) contenant un gaz comprimé tel que de l'air ou de l'azote et peut être mis en communication par l'opercule (72) avec l'élément gonflable (3). La communication entre le compartiment (75) et le compartiment pyrotechnique (74) est momentanément empêchée par un bouchon (76).

Dans une variante le compartiment contenant le gaz comprimé (75) peut être réalisé sous forme de plusieurs compartiments latéraux adjacents communiquant entre eux par des orifices (77).

Dans le cas où :
- la pression dans le réservoir est de 250 bars;
- le gaz de source pyrotechnique représente 50% du total;
- le foisonnement de la poudre pyrotechnique est de 2000/1 lors de son brûlage;
- le volume de gaz nécessaire au gonflage est de 50 litres dans les conditions normales de pression;
- le nombre d'éléments gonflables est de 8 pour l'ensemble de l'appareil;
alors, les volumes de gaz comprimé et de poudre pyrotechnique sont respectivement de l'ordre de 100 et 12 cm3 dans le cas où l'on veut effectuer le gonflage de l'ensemble des éléments avec une seule capsule. Enfin, le mélange de gaz décomprimé et donc très froid, et de gaz chaud provenant de la combustion de la poudre, permet une température adéquate du gaz de gonflage.

Dans le cas où l'on veut effectuer le gonflage de chaque élément par une capsule, celle-ci utilise alors un volume d'air de 12,5 cm3 et un volume de poudre pyrotechnique de 1,6 cm3. Dans cette variante, les capsules de gonflage ont des dimensions inférieures en hauteur à 5 cm, en largeur à 4 cm et en épaisseur à 1,6 cm et se logent donc facilement tout autour du dispositif permettant de protéger la personne.

Le fait que chaque élément gonflable comporte une capsule présente l'avantage d'avoir un chemin très court de gonflage et donc d'assurer un gonflage dans un temps très inférieur à 0,3 secondes. De cette façon l'appareil assure une protection efficace lors des chutes.

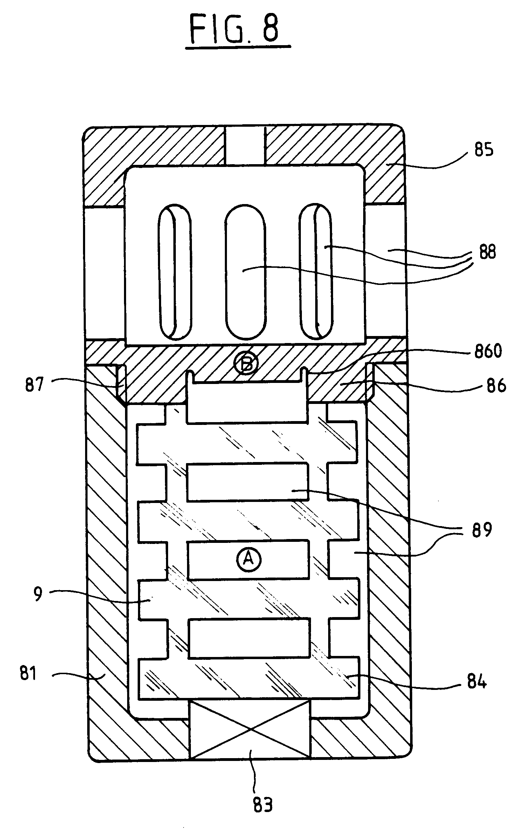

Un autre mode de réalisation d'une capsule de gonflage est représenté à la figure 8. La capsule métallique (81) comporte un compartiment inférieur étanche (A) fermé par un bouchon (86) résistant à la pression et dont la partie supérieure (85) est pourvue d'ouies (88). Cette partie supérieure (85) forme un logement creux au-dessus de la chambre dont une portion, située au-dessus des ouies (88) est destinée à accueillir un bouchon (86) après son cisaillement. Le compartiment (A) est rempli, d'une part d'éléments de poudre pyrotechnique agglomérée (84) dont la forme est telle qu'ils ne remplissent qu'une partie du compartiment (A) et que la poudre est répartie dans l'ensemble du volume du compartiment et d'autre part de gaz liquéfié (89) dans les conditions de température (température ambiante) et de pression de la capsule qui remplit l'espace laissé libre par la poudre, cette pression étant variable suivant le gaz liquéfié considéré, mais restent inférieure à 100 bars. Le compartiment (A) comprend à sa base une amorce pyrotechnique électrique (83). Le bouchon (86) comporte une rainure circulaire de cisaillement (860). Si la pression monte dans le compartiment A, on atteindra une valeur telle que la poussée sur le centre du bouchon (B) provoquera sa rupture suivant la rainure circulaire (860) et donc son éjection vers le logement creux (85). La capsule est installée sur l'enceinte à gonfler de telle sorte que la partie supérieure (85), y compris les ouies (88), soient à l'intérieur de l'enceinte.

En fonctionnement, lorsque le contact (5) est fermé, la tension aux bornes des amorces (73) ou (83) est nulle et il n'y a pas de mise en service du gonflage. Lorsque le contact (5) s'ouvre, si aucun des contacts (51, 52) de sécurité n'a été préalablement fermé, le circuit de détection (6) commande, après une temporisation éventuelle de 0,2 seconde, par le circuit (63), la conduction de l'interrupteur électronique (60) qui met ainsi en liaison la pile (50) avec les amorces pyrotechniques (73, 83). Ces dernières étant alors alimentées électriquement sont mises à feu. La poudre pyrotechnique (74) est alors mise à feu et dégage du gaz sous pression qui, dans un premier temps, éjecte le bouchon (76, 86).

Dans le cas de la figure 6, l'enveloppe métallique (71), et en particulier son alvéole centrale (72), monte en pression jusqu'à atteindre la pression de rupture de l'opercule (72) qui éclate, permettant l'alimentation de l'élément gonflable (3).

La capsule de gonflage de la figure 8 fonctionne de la manière suivante. Après la mise à feu de l'amorce pyrotechnique (83), la poudre pyrotechnique brûle en dégageant de la chaleur qui vaporise le gaz liquéfié, et en dégageant du gaz qui fait monter la pression dans le compartiment (A). Lorsque la pression de cisaillement du bouchon (86) est atteinte, celui-ci est éjecté et prend place à la partie supérieure (85) de la capsule. Le gaz, mélange de gaz de combustion de la poudre et de gaz liquéfié vaporisé, s'échappe alors par les ouies latérales et gonfle l'enceinte. En dosant correctement les quantités de poudre pyrotechnique et de gaz liquéfié, on peut obtenir un équilibrage des quantités de chaleur dégagées par la poudre et les quantités de chaleurs absorbées par le gaz liquéfié pour se vaporiser et se détendre. On obtient donc un gaz très proche de la température ambiante. Le gaz liquéfié peut être, soit du dioxyde de carbone (CO2) soit encore du gaz utilisé dans les circuits froids des réfrigérateurs (C.F.C. ou substitus modernes non polluants).

Lorsque la personne touche le sol à la fin de sa chute, les boudins (3, fig 7) sont gonflés et assurent la protection du col du fémur et de l'os du bassin.

Bien évidemment l'appareil est pourvu de moyens permettant le dégonflage après que le rôle de protection ait été rempli puisque l'amortissement de la chute ne dure que très peu de temps, de l'ordre de quelques dizièmes de seconde au maximum. Un dégonflage de la ceinture, ne nuit donc pas à son efficacité. Une étanchéité absolue n'est donc pas nécessaire, ce qui facilite sa fabrication.

On peut à cet effet prévoir un ou plusieurs bouchons de dégonflages. Mais on peut y adjoindre un dégonflage automatique : le tissu extérieur de la gaine est percé de trous calibrés de très petit diamètre. Le débit de fuite est négligeable pendant le fonctionnement et la période d'amortissement de la chute (de l'ordre de la seconde) et il se produit ensuite un dégonflage progressif en quelques dizaines de secondes. De même, une soupape de sécurité du type de celle repréentée à la figure 10 peut être prévue.

Dans le cas où les interrupteurs (51,52) sont fermés, l'ouverture de l'interrupteur (5), suite à une détection d'accélération n'est pas détecté par le circuit électronique (63) et ne déclenche pas la mise en conduction de l'interrupteur électronique (60). Ces interrupteurs (51,52) empêchent l'alimentation des amorces (73) et le gonflage du système lorsque, par exemple, la ceinture est enlevée ou lorsque l'utilisateur passe en position couchée.

Dans une variante l'enveloppe intérieure de l'élément support (1-10), conçue pour épouser la forme du corps est doublée, au contact de la peau, par un matériau confortable, amovible ou non.

De même dans une variante de l'appareil, celui-ci peut être incorporé de façon amovible ou non, à des vêtements.

D'autres modifications à la portée de l'homme de métier font également partie de l'esprit de l'invention, tels que des dispositifs d'amorçage à percussion et des accéléromètres mécaniques. Dans une variante l'élément de gonflage unique est relié par un conduit de gaz vers l'ensemble des éléments gonflables. Dans ce cas, un seul élément de gonflage permet le gonflage de tous les éléments disposés à la périphérie de l'élément support en forme de jupe. Dans une telle variante, chacun des éléments gonflables (3) communique avec les éléments adjacents par des orifices de communication, de façon à permettre le gonflage à partir de la réserve unique de gaz sous pression. Dans le cas de la variante où il existe une seule capsule de gonflage alimentant tous les éléments gonflables, cette capsule est reliée à un tuyau par exemple par un raccord à vis que l'on viendra fixer sur l'orifice (720) fileté proche de l'opercule (72, fig 6). Ainsi, un percuteur déclenché par un accéléromètre mécanique ou électrique peut percer l'opercule de fermeture d'un compartiment contenant un gaz comprimé de gonflage. De même, une électrovanne commandée par l'accéléromètre peut libérer le gaz comprimé contenu dans une enceinte.

## Revendications

1. Appareil préventif de fractures du col du fémur pour les personnes agées, comprenant un élément support (1,10) fixé de façon amovible, d'éléments de protection gonflables (3) , l'élément support (1, 10) étant fixé à la taille; les éléments gonflables (3) étant constitués par une pluralité d'éléments à soufflets, qui une fois gonflés, prennent une forme de boudin, ces éléments gonflables (3) étant solidaires de l'élément support (1, 10) et adjacents entre eux de façon à couvrir en permanence une zone s'étendant substantiellement de la taille jusqu'à mi-cuisse; caractérisé en ce que :
- l'appareil est muni de moyens de glonflage comprenant des capsules de gonflage (71) comportant une amorce pyrotechnique (73) à déclenchement électrique, les moyens de gonflage effectuant le gonflage de la pluralité des éléments gonflables (3) en un temps inférieur à 0,3 secondes ;
- des moyens de déclenchement du gonflage (6) et de détection des conditions d'une chute (5) sont disposés sur le devant de l'abdomen, les moyens de déclenchement du gonflage (6) comprenant un contact électrique (60) mettant en relation ou non une source électrique (50) avec chacune des amorces pyrotechniques (73) des capsules de gonflage (71) des éléments gonflables (3) et les moyens de détection des conditions d'une chute étant constitués d'un accéléromètre (5) se déclenchant à partir d'un seuil d'accélération compris entre 0,1 et 0,4 g, cet accéléromètre (5) commandant le contact électrique (60) pour mettre en relation ou non la source électrique (50) avec chacune des amorces pyrotechniques (73) des capsules de gonflage (71) des éléments gonflables (3).

2. Appareil selon la revendication 1, caractérisé en ce qu'il comporte une temporisation de déclenchement des moyens de gonflage (71) de l'ordre de 0,2 seconde et en ce que les moyens de gonflage (71) effectuent le gonflage en un temps inférieur à 0,1 seconde.

3. Appareil selon la revendication 2, caractérisé en ce que les éléments gonflables (3) comportent chacun une soupape de sécurité (33, 34) à fort débit.

4. Appareil selon la revendication 3, caractérisé en ce que chaque soupape à fort débit est constituée d'une fente dans un matériau plastique dont un bord (33) a une forme de jonc cylindrique (330) et l'autre (34) une forme de gorge cylindrique (340).

5. Appareil selon la revendication 1 ou 3, caractérisé en ce que les éléments gonflables (3) forment une jupe.

6. Appareil selon la revendication 1 ou 3, caractérisé en ce que les éléments gonflables couvrent la partie arrière du bassin jusqu'au bas des muscles fessiers et sur le côté des hanches descendent jusqu'à mi-cuisse.

7. Appareil selon la revendication 6, caractérisé en ce que les éléments gonflables descendant jusqu'à mi-cuisse sont reliés entre eux par un élément de fixation (31, 32) à la cuisse.

8. Appareil selon la revendication 1 ou 3, caractérisé en ce que les éléments gonflables constituent un cuissard ouvert sur la partie avant basse du bassin.

9. Appareil selon la revendication 1 ou 3, caractérisé en ce que chaque élément gonflable (3) comporte une capsule de gonflage amovible, comportant dans une enveloppe (71) un premier compartiment (75, A) communiquant avec l'élément gonflable (3) par un opercule (72, 86) et contenant un gaz comprimé ou liquéfié et contenant une charge de poudre pyrotechnique (74, 89) mise à feu par une amorce pyrotechnique (73, 83).

10. Appareil selon la revendication 9, caractérisé en ce que la charge de poudre pyrotechnique (74) est placée dans un second compartiment (74) communiquant avec le premier (75) par un bouchon (76) à faible résistance.

11. Appareil préventif de fractures du col du fémur pour les personnes agées, comprenant un élément support (1, 10), fixé de façon amovible ; d'éléments de protection gonflables (3), l'élément support (1, 10) étant fixé à la taille ; les éléments gonflables (3) étant constitués par une pluralité d'éléments à soufflets, qui, une fois gonflés, prennent une forme de boudin, ces éléments gonflables (3) étant solidaires de l'élément support (1, 10) et adjacents entre eux de façon à couvrir en permanence une zone s'étendant substantiellement de la taille jusqu'à mi-cuisse ; caractérisé en ce que :
- l'appareil est muni de moyens de gonflage comprenant une enceinte contenant un gaz comprimé, les moyens de gonflable effectuant le gonflage de la pluralité des éléments gonflables (3) en un temps inférieur à 0,3 seconde ;
- des moyens de déclenchement du gonflage et de détection des conditions d'une chute sont disposés sur le devant de l'abdomen, les moyens de déclenchement du gonflage comprenant un percuteur ou une électrovanne destiné à libérer un gaz comprimé enfermé dans l'enceinte et les moyens de détection des conditions d'une chute étant constitués d'un accéléromètre (5) se déclenchant à partir d'un seuil d'accélération compris entre 0,1 et 0,4g, cet accéléromètre (5) commandant le percuteur ou l'électrovanne de façon à libérer le gaz comprimé enfermé dans l'enceinte.

12. Appareil selon une des revendications précédentes, caractérisé en ce que l'élément support (1,10) comporte à l'intérieur une doublure , amovible ou non, en matériau confortable.

13. Appareil selon une des revendications précédentes, caractérisé en ce qu'il est incorporé et rendu solidaire, de façon amovible ou non, de l'intérieur de vêtements.

14. Appareil selon une des revendications précédentes caractérisé en ce qu'il est équipé d'un contact électrique (52) actionnable manuellement pour condamner temporairement la commande par le détecteur de seuil d'accélération du contact électrique mettant en relation une source électrique (50) avec chacune des amorces pyrotechniques (73) à déclenchement électrique des capsules de gonflage des éléments gonflables.

15. Appareil selon la revendication 14, caractérisé en ce que le contact électrique (52) est constitué par une des agrafes permettant de boucler l'élément support autour de la taille.

16. Appareil selon une des revendications précédentes caractérisé en ce qu'il est équipé d'un contact électrique (51) détectant les inclinaisons du corps supérieures à 60°, lequel contact électrique condamne la commande par le détecteur de seuil d'accélération du contact électrique mettant en relation une source électrique avec chacune des amorces pyrotechniques (73) à déclenchement électrique des capsules de gonflage (71) des éléments gonflables (3).

17. Procédé de prévention des fractures du col du fémur utilisant un élément gonflable pour couvrir en permanence par l'intermédiaire de l'élément gonflable une zone s'étendant substantiellement de la taille jusqu'à mi-cuisse et caractérisé en ce qu'il consiste à :
- détecter les conditions d'une chute ;
- retarder le déclenchement du gonflage de l'ordre de 0,2 seconde ;
- gonfler l'élément en un temps inférieur à 0,1 seconde ;
- utiliser la surpression due au contact avec la surface pouvant occasionner la fracture pour déclencher une soupape de sécurité prévue sur chaque élément gonflable dont le rôle est de déclencher pour une valeur relativement précise de la pression et d'assurer un fort débit instantané pour des pressions différentielles modérées.

## Claims

1. Device for preventing fractures of the neck of the femur of elderly persons and including a fixed support element (1, 10) able to be moved, inflatable protective elements (3), the support element (1, 10) being fixed to the waist, the inflatable elements (3) being formed of a plurality of bellows elements which, when once inflated, exhibit the shape of a sausage, these inflatable elements (3) being integral with the support element (1, 10) and adjacent to one another so as to continuously cover a zone extending substantially from the waist up to the mid-thigh region, wherein :
- the device is provided with inflating means including inflating capsules (71) comprising a pyrotechnic percussion cap (73) with electric triggering, the inflating means inflating the plurality of the inflatable elements (3) in less than 0.3 seconds;
- means for triggering inflation (6) and detecting the circumstances of a fall (5) are disposed on the front of the abdomen, the inflation triggering means (6) including an electric contact (60) possibly placing in relation an electric source (50) with each of the pyrotechnic percussion caps (73) of the inflating capsules (71) of the inflatable elements (3) and the means for detecting the circumstances of a fall being formed of an accelerometer (5) being triggered from an acceleration threshold of between 0.1 and 0.4 g, this accelerometer (5) ordering the electric contact (60) to possibly place in relation the electric source (50) with each of the pyrotechnic percussion caps (73) of the inflating capsules (71) of the inflatable elements (3).

2. Device according to claim 1, wherein it comprises a triggering delay time of the inflating means (71) of about 0.2 seconds and wherein the inflating means (71) carry out inflation in less than 0.1 seconds.

3. Device according to claim 2, wherein the inflatable elements (3) each comprise a slow flowrate safety valve (33, 34).

4. Device according to claim 3, wherein each slow flowrate valve is constituted by a slit in a plastic material whose edge (33) has a cylindrical cane shape (330) and the other (34) a cylindrical throat shape (340).

5. Device according to claim 1 or 3, wherein the inflatable elements form a skirt.

6. Device according to claim 1 or 3, wherein the inflatable elements cover the rear portion of the pelvis as far as the bottom of the gluteal or buttock muscles and on the side of the hips down to the mid-thigh region.

7. Device according to claim 6, wherein the inflatable elements going down to the mid-thigh region are interconnected by a fixing element (31, 32) to the thigh.

8. Device according to claim 1 or 3, wherein the inflatable elements constitute a bucket open on the front bottom portion of the pelvis.

9. Device according to claim 1 or 3, wherein each inflatable element (3) comprises a movable inflating capsule comprising in a casing (71) one first compartment (75, A) communicating with the inflatable element (3) via a cover (72, 86) and containing a compressed or liquified gas and containing a pyrotechnic powder charge (74, 89) ignited by a pyrotechnic percussion cap (73, 83).

10. Device according to claim 9, wherein the pyrotechnic powder charge (74) is placed in a second compartment (74) communicating with the first one (75) via a low resistance plug (76).

11. Device for preventing fractures of the neck of the femur of elderly persons and including a fixed support element (1, 10) able to be moved, inflatable protective elements (3), the support element (1, 10) being fixed to the waist, the inflatable elements (3) being constituted by a plurality of bellows elements which, once inflated, take the shape of a sausage, these inflatable elements (3) being integral with the support element (1, 10) and adjacent to one another so as to continuously cover a zone extending substantially from the waist to the mid-thigh region, wherein :
- the device is provided with inflating means including a chamber containing a compressed gas, the inflating means inflating the plurality of inflatable elements (3) is less than 0.3 seconds;
- means for triggering inflation and detecting the circumstances of a fall are disposed on the front of the abdomen, the means triggering inflation including a striking pin or electrovalve intended to free a compressed gas contained in the chamber and means for detecting the circumstances of a fall being constituted by an accelerometer (5) being triggered from an acceleration threshold of between 0.1 and 0.4g, this accelerometer (5) ordering the striking pin or electrovalve to free the compressed gas contained in the chamber.

12. Device according to any one of the preceding claims, wherein the support element (1, 10) internally comprises a lining, possibly movable, made of a soft material.

13. Device according to any one of the preceding claims, wherein it is rendered integral inside clothing and may, if need be, be able to be moved.

14. Device according to any one of the preceding claims, wherein it is equipped with a manually activatable electric contact (52) so as to temporally interrupt the command by the acceleration threshold of the electric contact placing an electric source (50) in relation with each of the pyrotechnic percussion caps (73) with electric triggering of the inflating capsules of the inflatable elements.

15. Device according to claim 14, wherein the electric contact (52) is constituted by one of the hooks able to fasten the support element around the waist.

16. Device according to any one of the preceding claims, wherein it is equipped with an electric contact (51) detecting the slantings of the body of more than 60 degrees, said electric contact interrupting the order by the acceleration threshold of the electric contact placing an electric source in relation with each of the pyrotechnic percussion caps (73) with electric triggering of the inflating capsules (71) of the inflatable elements (3).

17. Method for preventing fractures of the neck of the femur using an inflatable element to continuously cover by means of the inflatable element a zone substantially extending from the waist down to the mid-thigh region and wherein it consists of :
- detecting the circumstances of a fall ;
- delaying the triggering of inflating by about 0.2 seconds ;
- inflating the element in less than 0.1 seconds ;
- using the excess pressure due to the contact with the surface able to cause the fracture so as to trigger a safety valve provided on each inflatable element whose rôle is to trigger, for a relatively precise value of the pressure, and ensure a high instantaneous flowrate for moderate differential pressures.

## Patentansprüche

1. Gerät zur Vorbeugung gegen Schenkelhalsbrüche bei älteren Personen, bestehend aus einem abnehmbar angebrachten Trägerteil (1, 10), aufblasbaren Schutzelementen (3), die aus einer Mehrzahl von balgartigen, in aufgeblasenem Zustand wulstförmigen Elementen bestehen, die fest mit dem Trägerteil (1, 10) verbunden und aneinanderliegend angeordnet sind, sodaß sie durchgehend einen Bereich bedecken, der sich im Wesentlichen von der Taille bis in halbe Höhe des Oberschenkels erstreckt, **dadurch gekennzeichnet, daß** das Gerät mit folgenden Teilen versehen ist:
- Aufblasmittel, die aus Aufblaspatronen (7) bestehen, welche einen elektrisch auslösbaren Sprengzünder (73) besitzen, sodaß die Gesamtzahl der aufblasbaren Elemente (3) innerhalb von 0,3 Sekunden aufgeblasen werden,
- Mittel zum Auslösen des Aufblasens (6) und Mittel zum Erkennen der Umstände eines Stürzens der Person (5), die auf der Bauchseite angebracht sind, wobei die Mittel zum Auslösen des Aufblasens (6) einen elektrischen Kontakt (60) zur Bildung einer Verbindung zwischen einer Stromquelle (50) und jedem einzelnen der Sprengzünder (73) der an den aufblasbaren Elementen (3) angebrachten Aufblaspatronen (71) besitzen und die Mittel zum Erkennen der Umstände eines Stürzens der Person aus einem Beschleunigungsmesser (5) bestehen, der den elektrischen Kontakt (60) zur Bildung einer Verbindung zwischen einer Stromquelle (50) und jedem einzelnen der Sprengzünder (73) der an den aufblasbaren Elementen (3) angebrachten Aufblaspatronen (71) herstellt.

2. Gerät nach Patentanspruch 1, dadurch gekennzeichnet, daß es eine Auslöseverzögerung der Aufblaspatronen (71) von annähernd 0,2 Sekunden besitzt und die Aufblaspatronen (71) das Aufblasen innerhalb von 0,1 Sekunden bewirken.

3. Gerät nach Patentanspruch 2, dadurch gekennzeichnet, daß die aufblasbaren Elemente (3) jeweils ein Sicherheitsventil (33, 34) mit hohem Durchsatz besitzen.

4. Gerät nach Patentanspruch 3, dadurch gekennzeichnet, daß jedes Sicherheitsventil mit hohen Durchsatz aus einem Spalt in einem Kunststoffmaterial besteht, bei dem Rand (33) die Form eines zylindrischen Wulstes (330) und der andere Rand (34) die Form einer zylindrischen Hohlkehle (340) besitzt.

5. Gerät nach Patentanspruch 1 oder 3, dadurch gekennzeichnet, daß die aufblasbaren Elemente (3) einen Rock bilden.

6. Gerät nach Patentanspruch 1 oder 3, dadurch gekennzeichnet, daß die aufblasbaren Elemente am hinteren Bereich des Beckens bis zur Unterseite der Gesäßmuskeln und seitlich der Hüften bis zur halben Höhe der Oberschenkel hinunterreichen.

7. Gerät nach Patentanspruch 6, dadurch gekennzeichnet, daß die aufblasbaren Elemente, die bis zur halben Höhe der Oberschenkel hinunterreichen gemeinsam durch eine Trägerelement (31, 32) mit dem Oberschenkel verbunden sind.

8. Gerät nach Patentanspruch 1 oder 3, dadurch gekennzeichnet, daß die aufblasbaren Elemente eine Beinschiene bilden, die aus der Unterseite vorn offen ist.

9. Gerät nach Patentanspruch 1 oder 3, dadurch gekennzeichnet, daß jedes aufblasbare Element (3) über eine abnehmbare Aufblaspatrone verfügt, die in einer Hülle (71) eine erste Kammer (75, A) besitzt, die über ein Innenhütchen (72, 86) mit dem aufblasbaren Element (3) verbunden ist und ein verdichtetes bzw. flüssiges Gas sowie eine Ladung entzündbaren Pulvers (74, 89) enthält, das durch einen Sprengzünder (73, 83) entzündet wird.

10. Gerät nach Patentanspruch 9, dadurch gekennzeichnet, daß die Ladung entzündbaren Pulvers (74) in einer zweiten Kammer (74) untergebracht ist, deren Verbindung mit der ersten Kammer (75) durch einen Stopfen (76) mit geringer Festigkeit unterbunden ist.

11. Gerät zur Vorbeugung gegen Schenkelhalsbrüche bei älteren Personen, bestehend aus einem abnehmbar angebrachten Trägerteil (1, 10), aufblasbaren Schutzelementen (3), die aus einer Mehrzahl von balgartigen, in aufgeblasenem Zustand wulstförmigen Elementen bestehen, die fest mit dem Trägerteil (1, 10) verbunden und aneinanderliegend angeordnet sind, sodaß sie durchgehend einen Bereich bedecken, der sich im Wesentlichen von der Taille bis in halbe Höhe des Oberschenkels erstreckt, **dadurch gekennzeichnet, daß**
- das Gerät mit Aufblasmitteln versehen ist, die einen Druckgasbehälter besitzen und mit denen die Gesamtzahl der aufblasbaren Elemente (3) innerhalb von 0,3 Sekunden aufgeblasen werden,
- Mittel zum Auslösen des Aufblasens und Mittel zum Erkennen der Umstände eines Stürzens der Person auf der Bauchseite angebracht sind, wobei die Mittel zum Auslösen des Aufblasens einen Schlagbolzen oder ein Elektroventil besitzen, mit dem das in dem Druckgasbehälter befindliche Gas freigesetzt wird und die Mittel zum Erkennen der Umstände eines Stürzens der Person aus einem Beschleunigungsmesser (5) bestehen, der bei einem Schwellenwert zwischen 0,1 und 0,4 g ausgelöst wird und den Schlagbolzen oder das Elektroventil auslöst, mit dem das in den Druckgasbehälter befindliche Gas freigesetzt wird.

12. Gerät nach einem der oben beschriebenen Patentansprüche, dadurch gekennzeichnet, daß das Trägerteil (1, 10) auf der Innenseite ein abnehmbares oder fest angebrachtes Futter aus bequemen Material besitzt.

13. Gerät nach einem der oben beschriebenen Patentansprüche, dadurch gekennzeichnet, daß es abnehmbar oder fest in einem Kleidungsstück angebracht ist.

14. Gerät nach einem der oben beschriebenen Patentansprüche, dadurch gekennzeichnet, daß es mit einem elektrischen Kontakt (52) versehen ist, der von Hand ausgelöst werden kann, im die Herstellung des elektrischen Kontaktes zur Bildung einer Verbindung zwischen einer Stromquelle (50) und jedem einzelnen der Sprengzünder (73) der an den aufblasbaren Elementen angebrachten Aufblaspatronen zeitweilig zu unterbinden.

15. Gerät nach Patentanspruch 14, dadurch gekennzeichnet, daß der elektrische Kontakt (52) aus einer der Klammern besteht, mit denen das Trägerteil um die Taille befestigt wird.

16. Gerät nach einem der oben beschriebenen Patentansprüche, dadurch gekennzeichnet, daß es mit einem elektrischen Kontakt (51) versehen ist, der eine Schrägneigung des Körpers über 60° erkennt und das Auslösen des elektrischen Kontaktes zur Bildung einer Verbindung zwischen einer Stromquelle und jedem einzelnen der Sprengzünder (73) der an den aufblasbaren Elementen (3) angebrachten Aufblaspatronen (71) unterbindet.

17. Verfahren zur Vorbeugung gegen Schenkelhalsbrüche unter Verwendung eines aufblasbaren Elementes, das durchgehend einen Bereich abdeckt, der im Wesentlichen von der Taille bis zur halben Höhe des Oberschenkels reicht, dadurch gekennzeichnet, daß durch es darin besteht,
- die Umstände eines Stürzens der Person zu erkennen,
- die Auslösung des Aufblasens um annähernd 0,2 Sekunden zu verzögern,
- das aufblasbare Element innerhalb von 0,1 Sekunden aufzublasen,
- den Überdruck, der durch die Berührung mit der den Schenkelhalsbruch bewirken könnenden Oberfläche verursacht wird, zum Auslösen eines an jedem aufblasbaren Element vorgesehenen Sicherheitsventils zu benützen, dessen Rolle darin besteht, bei einem relativ genauen Druckwert ausgelöst zu werden und einen sofortigen hohen Durchsatz zum Erreichen eines gemäßigten Differentialdruckes zu gewährleisten.
